# EUROPÄISCHE PATENTSCHRIFT

(11) **EP 2 268 359 B1**
(45) Veröffentlichungstag und Bekanntmachung des Hinweises auf die Patenterteilung: **12.09.2012**
(21) Anmeldenummer: 09735486.4
(22) Anmeldetag: 21.04.2009
(51) Int. Cl.: A61N 5/00, G21K 1/08, G01T 1/28

(54) **TEILCHENSTRAHL-THERAPIEANLAGE UND VERFAHREN ZUM FÜHREN EINES STRAHLS GELADENER TEILCHEN IN EINER TEILCHENSTRAHL-THERAPIEANLAGE**
PARTICLE BEAM THERAPY SYSTEM AND METHOD FOR GUIDING A BEAM OF CHARGED PARTICLES IN A PARTICLE BEAM THERAPY SYSTEM
DISPOSITIF THÉRAPEUTIQUE À FAISCEAU DE PARTICULES ET PROCÉDÉ DE GUIDAGE D'UN FAISCEAU DE PARTICULES CHARGÉES DANS UN DISPOSITIF THÉRAPEUTIQUE À FAISCEAU DE PARTICULES

(30) Priorität: 21.04.2008 DE 102008020039
(43) Veröffentlichungstag der Anmeldung: 05.01.2011
(73) Patentinhaber: Varian Medical Systems Particle Therapy GmbH, 51429 Bergisch Gladbach (DE)
(72) Erfinder: DREES, Jürgen, 42287 Wuppertal (DE); PIEL, Helmut, 42287 Wuppertal (DE)
(74) Vertreter: Kirchner, Veit
(86) Internationale Anmeldenummer: PCT/EP2009/054747
(87) Internationale Veröffentlichungsnummer: WO 2009/130220

(56) Entgegenhaltungen:
- EP-A- 1 876 870
- WO-A-2007/045076
- PETERS A ET AL: "BEAM DIAGNOSTICS FOR THE HEAVY ION CANCER THERAPY FACILITY" AIP CONFERENCE PROCEEDINGS, AMERICAN INSTITUTE OF PHYSICS, NEW YORK, US, Nr. 546, 8. Mai 2000 (2000-05-08), Seiten 519-526, XP008003048 ISSN: 0094-243X
- FORCK P ET AL: "A scintillator based halo-detector for beam position monitoring" EUROPEAN PARTICLE ACCELERATOR CONFERENCE. EPAC, XX, XX, Bd. 3, 14. Juni 1997 (1997-06-14), Seiten 2644-2646, XP009120834 ISBN: 978-0-7503-0386-6
- EICKHOFF H ET AL: "HICAT- the German hospital-based light ion cancer therapy project" PROCEEDINGS OF THE 2003 PARTICLE ACCELERATOR CONFERENCE. PAC 2003. PORTLAND, OR, MAY 12 - 16, 2003; [PARTICLE ACCELERATOR CONFERENCE], NEW YORK, NY : IEEE, US, Bd. 1, 12. Mai 2003 (2003-05-12), Seiten 694-698, XP010700523 ISBN: 978-0-7803-7738-7
- [Online] 2006, , XP002539474 Gefunden im Internet: URL:http://web.archive.org/web/20061024055 507/http://www.detectors.saint-gobain.com/ Data/Element/Node/Category/Category_edit.a sp?ele_ch_id=C0000000000000001855&Lang=US> [gefunden am 2009-07-30] das ganze Dokument
- [Online] 2005, , XP002539473 Gefunden im Internet: URL:http://web.archive.org/web/20060504083 702/www.detectors.saint-gobain.com/Media/D ocuments/S0000000000000001004/SGC+BC400-40 4-408-412-416+Data+Sheet+0105.pdf> [gefunden am 2009-07-30] das ganze Dokument
- [Online] 2007, , XP002539475 Gefunden im Internet: URL:http://www.detectors.saint-gobain.com/ Media/Documents/S0000000000000001004/SGC_G lass_Scintillator_Data_Sheet.pdf> [gefunden am 2009-07-30] das ganze Dokument
- EICKHOFF H: "Controls and Beam Diagnostics for Therapy-Accelerators" PROCEEDINGS OF THE DIPAC 1999 - CHESTER, UK, 1999, Seiten 24-27, XP002561601 Chester, UK
- DÖLLING R: "Progress of the Diagnostics at the PROSCAN Beam Lines" PROCEEDINGS OF THE DIPAC, 2007, VENICE, ITALY, 2007, Seiten 361-363, XP002561602 Venice, Italy
- DÖLLING R: "Profile, current, and halo monitors of the PROSCAN beam lines" ELEVENTH BEAN INSTRUMENTATION WORKSHOP 2004 3-6 MAY 2004 KNOXVILLE, TN, USA, Nr. 732, 2004, Seiten 244-252, XP002561603 AIP Conference Proceedings AIP USA ISSN: 0094-243X & DATABASE INSPEC [Online] THE INSTITUTION OF ELECTRICAL ENGINEERS, STEVENAGE, GB; 2004, Database accession no. 8350436
- DÖLLING R: "Ionisation Chambers and Secondary Emission Monitors at the PROSCAN Beam Lines" (PROCEEDINGS OF THE ) BEAM INSTRUMENTATION WORKSHOP 2006: TWELFTH WORKSHOP, 2006, Seiten 271-280, XP002561604
- PETER STREHL ED - PETER STREHL: "CHAPTER 2: Beam Intensity Measurements" BEAM INSTRUMENTATION AND DIAGNOSTICS (BOOK SERIES: PARTICLE ACCELERATION AND DETECTION), SPRINGER BERLIN / HEIDELBERG, 1. Januar 2006 (2006-01-01), Seiten 11-70, XP009127184 ISBN: 978-3-540-26401-9 [gefunden am 2006-06-18]

## Beschreibung

Die Erfindung betrifft eine Teilchenstrahl-Therapieanlage mit einer Beschleunigereinrichtung zur Bereitstellung eines Strahls geladener Teilchen, mit einer Strahlführungseinheit, welche den Strahl ausgehend von der Beschleunigereinrichtung einem Behandlungsort zuführt, mit wenigstens einem entlang des Strahlwegs angeordneten Strahleigenschafts-Monitor, und mit einer mit dem Strahleigenschafts-Monitor und der Strahlführungseinheit kommunizierenden Kontrolleinheit, welche Abweichungen der Isteigenschaften von den Solleigenschaften des Strahls ermittelt und an Hand der Abweichungen Stellsignale zur Anpassung der Strahleigenschaften an die Strahlführungseinheit übermittelt. Die Erfindung betrifft weiterhin ein Teilchenstrahl-Korrekturverfahren.

Die Strahlentherapie mit geladenen Teilchen, beispielsweise Ionen in Form von Protonen, bietet gegenüber der bisher üblichen Strahlentherapie mit Photonen wesentliche Vorteile für Patienten mit bestimmten Krebserkrankungen. Insbesondere ist eine Bestrahlung mit Ionen, insbesondere Protonen, vorteilhaft, da diese erst am Ende ihres Laufwegs im zu bestrahlenden Gewebe, im so genannten Bragg-Peak, ihre maximale Ionisationsstärke, und damit einhergehend ihre größte Zerstörungskraft, beispielsweise für Tumorzellen, aufweisen. Auf diese Weise kann die Beeinträchtigung von gesundem Gewebe, welches dem zu behandelnden Gewebe im Laufweg des Strahls vorgelagert ist und von dem Strahl durchlaufen wird, reduziert werden.

Eine besonders wichtige und aufwändige Komponente des magnetoptischen Transportsystems zur Führung der Teilchen vom Beschleuniger bis zum Patienten besteht in einem als Gantry bezeichneten Traggestell. Dieses Traggestell lässt sich um eine, vorzugsweise horizontale, Rotationsachse drehen und ermöglicht dadurch die Bestrahlung des Tumorgewebes unter verschiedenen Winkeln innerhalb eines großen Winkelintervalls, gegebenenfalls bis zu 360°. Während einer Verdrehung der Gantry ändert sich die Lage der massigen Strahlführungsmagnete im Schwerefeld, so dass es durch die geänderten Gravitationskräfte zu einer geringfügigen, unvorhersehbaren Ortsabweichung der Strahlführungsmagnete gegenüber einer gewünschten Position kommen kann. In der Konsequenz kann dies erhebliche räumliche Abweichungen des Zentrums des Teilchenstrahls von der Sollposition bewirken, welche im weiteren Verlauf des Strahlwegs auch in Impulsabweichungen zutage treten können.

Die erfolgreiche Behandlung von Tumoren mit einem Teilchenstrahl, beispielsweise mittels des Raster-Scan-Therapieverfahrens, erfordert eine präzise Kenntnis der Strahleigenschaften, insbesondere der Strahlposition oder des Strahlimpulses, am Behandlungsort, d.h. am Isozentrum. Bereits relativ kleine Ortsabweichungen der Multipolmagnete der Strahlführungseinheit können insbesondere zu unerwünschten Ortsabweichungen des Teilchenstrahls am Isozentrum führen. Diese räumlichen Abweichungen des Teilchenstrahls können ein Mehrfaches der Ortsabweichungen der Multipolmagnete gegenüber der gewünschten Position betragen. Die beispielsweise rotationsbedingte Verschiebung eines als Fokussierelement verwendeten Quadrupolmagnets der Gantry von einem Millimeter kann beispielsweise zu einer räumlichen Abweichung des Strahls am Isozentrum von bis zu zehn Millimetern führen. Es ist dabei schwierig, Abweichungen der Strahlführungsmagnete bei der Drehung der Gantry im Rahmen der einzuhaltenden Toleranz, beispielsweise durch spezielle Befestigungsarten, vollständig zu vermeiden. Eine Korrektur ist daher erforderlich, um Behandlungsungenauigkeiten zu vermeiden.

Es ist, beispielsweise aus der Patentschrift EP 1 348 465 Bl, bekannt, Strahlabweichungen durch den Einsatz von Korrekturmagneten im Strahlführungssystem zu kompensieren. Nachteilig an den bekannten Anlagen ist jedoch, dass zur Erfassung der Istposition des Strahls Monitore verwendet werden, welche den Teilchenstrahl derart beeinflussen, dass der Transmissionsgrad des Strahls vom Beschleuniger bis zum Behandlungsort auf ein für die Behandlung untaugliches Maß herabgesetzt ist. Demzufolge muss die Anpassung der Istposition des Strahls an die gewünschte Sollposition zeitlich vor dem eigentlichen Behandlungsvorgang erfolgen. Bei Teilchenstrahl-Therapieanlagen, welche im Raster-Scan-Therapieverfahren betrieben werden, kann somit ein, insbesondere zeitlich, signifikanter Nachjustierungsaufwand entstehen.

Neben rotationsbedingten Einflüssen kann es überdies auch zu unvorhersehbaren Veränderungen der Strahlposition kommen, wenn im Raster-Scan-Therapieverfahren die Strahlenergie im Verlauf des Behandlungsvorgangs geändert wird, um beispielsweise den Energieeintrag in das zu behandelnde Gewebe und damit verbunden die Strahleindringtiefe zu variieren. Unvorhersehbare Veränderungen der Strahlposition können beispielsweise durch Hysteresephänomene bei der Variation des Erregerstroms in den Magnetfeldspulen der Elektromagnete der Strahlführungseinheit hervorgerufen werden. Unvorhergesehene Variationen des Feldes der Strahlführungsmagnete können auch durch Stromschwankungen der Netzgeräte der Magnete verursacht werden.

Die Veröffentlichung "BEAM DIAGNOSTICS FOR THE HEAVY ION CANCER THERAPY FACILITY", Peters et al., American Institute of Physics (AIP) Conference Proceedings Nr. 546, S. 519-526, New York, 8. Mai 2000, beschreibt verschiedene Strahl-Diagnostik-Monitore für die Strahlen des HICAT Systems, unter anderem ein nicht-destruktives Diagnostiksystem, das online zur Kontrolle des Strahls während der Patientenbehandlung und in allen anderen Fällen eingesetzt werden kann.

Die WO 2007/045076 betrifft die Integration einer "Magnetic Resonance Imaging" (MRI) Apparatur in die rotierende Gantry eines Strahlentherapie-Systems.

Die Veröffentlichung "A scintillator based halo-detector for beam position monitoring", Forck P. et al., European Particle Accelerator Conference (EPAC), Bd. 3, S. 2644-2636, 14. Juni 1997, beschreibt einen Halo-Detektor, welcher als Plastik-Szintillator außerhalb des Vakuums angeordnet ist.

Die Veröffentlichung "HICAT - the German hospital-based light ion cancer therapy project", Eickhoff H. et al., Proceedings of the 2003 Particle Accelerator Conference, PAC 2003, Portland, OR, 12.-16. Mai 2003, Bd. 1, S. 694-698, enthält eine Beschreibung von Messungen von Strahlposition und Winkel des von einem Synchrotron erzeugten Ionenstrahls.

Die Veröffentlichung "Controls and Beam Diagnostics for Therapy-Accelerators", Eickhoff H., Proceedings of the DIPAC 1999, Chester, UK, 1999, S. 24-27, beschreibt die Kontroll-und Strahl- Diagnostik für Therapie-Beschleuniger.

Die drei Veröffentlichungen von R. Dölling: "Progress of the Diagnostics at the PROSCAN Beam Lines", Proceedings of the DIPAC 2007, Venedig, Italien, 2007, S. 361-363, "Profile, current, and halo monitors of the PROSCAN beam lines", Eleventh Beam Instrumentation Workshop 2004, 3.-6. Mai 2004, Knoxville, TN, USA, Nr. 732, S. 244-252 und "Ionisation Chambers and Secondary Emission Monitors at the PROSCAN Beam Lines" (Proceedings of the) Beam Instrumentation Workshop 2006: Twelfth Workshop, 2006, S. 271-280 beschreiben einen permanent im Strahl befindlichen Positionsmonitor, der Protonen aus dem Halobereich des Strahls abgreift, um daraus den Strahlschwerpunkt zu bestimmen.

Hiervon ausgehend liegt der Erfindung die Aufgabe zu Grunde, eine Teilchenstrahl-Therapieanlage anzugeben, mit welcher eine präzise Strahlführung auch während eines Behandlungsvorgangs, insbesondere während der Durchführung eines Teilchenstrahl-Therapieverfahrens, gewährleistet werden kann. Ferner liegt der Erfindung die Aufgabe zu Grunde, ein geeignetes Teilchenstrahl-Korrekturverfahren anzugeben.

Gemäß einer ersten Lehre der vorliegenden Erfindung wird die Aufgabe durch eine Teilchenstrahl-Therapieanlage gemäß Patentanspruch 1 gelöst.

Der Erfindung liegt die Überlegung zu Grunde, eine sich automatisch regelnde Führung des Teilchenstrahls zur Kompensation der vorstehend beschriebenen Abweichungen der Strahleigenschaften auch während des Behandlungsvorgangs zur Verfügung zu stellen. Folglich können Abweichungen kompensiert werden, welche insbesondere bei einer während der Behandlung erforderlichen Rotation der Gantry und/oder bei Stromschwankungen der Netzgeräte und/oder bei einer während der Behandlung erforderlichen Variation der Energie des Teilchenstrahls auftreten. Das Maß an Beeinflussung des Strahls geladener Teilchen durch den Strahleigenschafts-Überwachungsprozess wird möglichst gering gehalten, um einen für die Behandlung ausreichenden Transmissionsgrad des Strahls von der Beschleunigereinrichtung bis zum Behandlungsort zu erzielen. Auf diese Weise kann das vor der Inbetriebnahme der Teilchenstrahl-Therapieanlage durchzuführende Mess- und Strahljustierungsprogramm eingeschränkt und - nach dem Start der Behandlungen - ein stabiler, unterbrechungsfreier Betrieb sichergestellt werden.

Dies kann dadurch erreicht werden, dass die unmittelbare Wechselwirkung des Strahlpositions-Monitors auf den Halobereich des Teilchenstrahls beschränkt wird. Der Halobereich kennzeichnet einen äußeren Randbereich des Strahls - betrachtet im Querschnitt in einer Richtung senkrecht zum Strahlweg. Wird die Verteilung der Leistungsdichte des Strahls im Querschnitt durch das Strahlzentrum, also das Strahlprofil, beispielsweise durch einen gaußkurvenförmigen Verlauf wiedergegeben (auch andere Verteilungen sind möglich), kann der Halobereich als der Randbereich definiert werden, in dem die Leistungsdichte gegenüber dem Leistungsdichtenmaximum im zentralen Teil des Strahls um eine bestimmtes Maß, beispielsweise mehrere Größenordnungen (wie etwa ein Tausendstel), reduziert ist. Auf diese Weise ist sichergestellt, dass die Teilchen im zentralen Teil des Strahls weitgehend unbeeinflusst - also weitgehend ohne Transmissionsverluste - dem Behandlungsort zugeführt werden können, wohingegen nur eine vergleichsweise geringe Anzahl an Teilchen am Rand des Strahls zur - gegebenenfalls verlustreichen - Eigenschaftsbestimmung herangezogen wird. Vorzugsweise ist die räumliche Ausdehnung des Strahlpositions-Monitors in Richtung des Strahlwegs sehr klein gehalten, um die Wechselwirkungsstrecke der Teilchen im Halobereich mit dem Material des Strahlpositions-Monitors gering zu halten.

Um während eines Behandlungsvorgangs eine schnelle Korrektur etwaiger Abweichungen vornehmen zu können, ist die Kontrolleinheit vorzugsweise zur zeitnahen Registrierung und Verarbeitung der Signale des Strahlpositions-Monitors ausgebildet. Falls die Messdaten des Strahlpositiöns-Monitors in analoger Form an die Kontrolleinheit übermittelt werden, beinhaltet dies deren zeitnahe digitale Aufbereitung beispielsweise in Form der Berechnung digitaler Stellsignale und deren Übermittlung an die Stahlführungseinheit und deren magnetoptische Elemente.

Basierend auf den Messungen von Strahlpositions-Monitoren berechnet eine spezielle in der Kontrolleinheit implementierte Computersoftware insbesondere die Orts- und Winkelabweichung des Zentrums des Teilchenstrahls am Isozentrum und die zur Korrektur erforderlichen magnetischen Feldstärken und Feldrichtungen der dafür vorgesehenen Multipolmagnete der Strahlführungseinheit. Für den Fall, dass mit der Teilchenstrahl-Therapieanlage ein Raster-Scan-Therapieverfahren ausgeführt wird, können zusätzlich die magnetischen Feldstärken und Feldrichtungen zur Einstellung der hinsichtlich Ort und Impuls gewünschten Isteigenschaften des Strahlzentrums berechnet werden, wie durch den zeitlichen Ablauf des Raster-Scan-Therapieverfahrens vorgegeben. Die entsprechenden Einstellungen erfolgen dabei über die Kontrolleinheit computergesteuert mit sehr geringer Zeitverzögerung.

Es ist möglich, wenigstens zwei Strahlpositions-Monitore entlang des Strahlwegs vorzusehen. Auf diese Weise kann eine Korrektur auf eine Abweichung von Ort und Winkel (mit anderen Worten Impuls) des Strahls geladener Teilchen in einer zum Strahlweg transversalen Koordinatenrichtung durchgeführt werden. Auch ist es möglich, entlang des Strahlwegs andere Monitore zur Überwachung des Strahls geladener Teilchen vorzusehen. Diese anderen Monitore können insbesondere unmittelbar hinter der Beschleunigereinrichtung und/oder unmittelbar vor dem Behandlungsort zum Einsatz kommen.

Der Strahlpositions-Monitor kann, gegebenenfalls mehrere Strahlpositionsmonitore können, die zum Strahlweg transversale Position des Strahls und/oder den zum Strahlweg transversalen Impuls des Strahls in wenigstens einer Dimension erfassen. Vorzugsweise wird der gesamte transversale Ausschnitt des Phasenraums der Teilchen durch den Strahlpositions-Monitor, oder die Strahlpositions-Monitore, erfasst. Auf diese Weise kann eine besonders verlässliche StrahleigenschaftsÜberwachung sichergestellt werden.

In einer vorteilhaften Ausgestaltung kann der Strahlpositions-Monitor eine zum Strahlweg orthogonale Detektionsfläche aufweisen, wobei die Detektionsfläche mit dem Strahlquerschnitt in dessen Halobereich überlappt. Durch die Beschränkung der Wechselwirkung der Teilchen mit dem Strahlpositions-Monitor an der Peripherie des Strahls im Halobereich kann eine Beeinflussung des Strahls, insbesondere eine Strahlaufweitung durch den Überwachungsprozess, sehr gering gehalten werden.

Die Kontrolleinheit kann derart eingerichtet sein, dass die Isteigenschaften des Strahls aus den Messungen des Strahlpositions-Monitors unter Einbeziehung von Kalibrationsdaten ermittelt werden. Die Kalibrationsdaten ermöglichen dabei insbesondere, das räumliche Zentrum des Strahls, welches mit dem Ort der höchsten Leistungsdichte im Falle eines symmetrischen Strahls übereinstimmen kann, an Hand der an der Peripherie des Strahls, beispielsweise im Halobereich, aufgenommenen Messdaten zu extrapolieren. Vorzugsweise wird zu diesem Zweck das Profil des Strahls in einer zum Strahlweg transversalen Ebene vor der ersten Inbetriebnahme - und aktualisierend gegebenenfalls zwischen einzelnen Betriebsperioden der Teilchenstrahl-Therapieanlage - vermessen, und diese Messdaten werden der Kontrolleinheit als Kalibrationsdaten zur Verarbeitung zur Verfügung gestellt. Ausgehend von einer aktuellen Messung der Strahleigenschaften an der Peripherie des Strahls kann dann auf das zwischen den Messpunkten befindliche Strahlprofil unter Berücksichtigung der Kalibrationsdaten mit hoher Genauigkeit zurückgeschlossen werden.

In vorteilhafter Weise sind zwei Strahlpositions-Monitore einander diametral gegenüberliegend an der Peripherie des Strahls angeordnet. Auf diese Weise kann der Strahleigenschafts-Überwachungsprozess mit hoher Verlässlichkeit ausgeführt werden, denn die beispielsweise durch eine Strahlpositionsverschiebung an einem Ort an der Peripherie des Strahls bewirkte Abschwächung eines Strahleigenschafts-Messsignals bewirkt gleichzeitig eine Messsignalverstärkung an einem diesem Ort diametral gegenüberliegenden Ort. Darüber hinaus kann die Güte der ermittelten Isteigenschaften des Strahls verbessert werden, weil die Kalibrationsdaten-Auswertung auf einer breiteren Messdatengrundlage fußt.

Der Strahlpositions-Monitor kann wenigstens einen Szintillationszähler aufweisen. Insbesondere können mehrere Szintillationszähler als Szintillationszählerarrays an der Peripherie des Teilchenstrahls angeordnet werden.

Das Szintillationsmaterial kann in Richtung des Strahlwegs eine Dicke von etwa 0,5 bis 2 mm aufweisen. Auf diese Weise können die Wechselwirkungsstrecke der Teilchen mit dem Strahlpositions-Monitor im Halobereich und damit die Beeinflussung der Strahleigenschaften weiter verringert werden. Insbesondere einer dem Transmissionsgrad abträglichen Strahlaufweitung lässt sich damit besser vorbeugen.

Der Strahlpositions-Monitor kann wenigstens eine, vorzugsweise zylindrische, Ionisationskammer aufweisen. Insbesondere können mehrere Ionisationskammern in Richtung des Strahlwegs in mehreren Monitorebenen angeordnet werden. Zylindrische Ionisationskammern weisen insbesondere eine drahtförmige Elektrode auf, um welche konzentrisch eine hohlzylinderförmige, auf unterschiedlichem elektrischen Potential gehaltene Gegenelektrode angeordnet ist (diese Art der Ausgestaltung wird im Englischen auch Straw Tube genannt). Prinzipiell kann der Strahlpositions-Monitor auch außerhalb des Vakuumbereichs der Strahlführungseinheit angeordnet werden. Diese Ausgestaltung bietet insbesondere Freiheiten in der Ausgestaltung und Anordnung der Strahlpositions-Monitore, welche nicht mehr durch den eingeschränkten Platz im Vakuumbereich limitiert sind.

In einer vorteilhaften Ausgestaltung ist der Strahlpositons-Monitor transversal zum Strahlweg beweglich. Auf diese Weise kann flexibel auf Abweichungen der Strahleigenschaften, insbesondere der Strahlposition reagiert, und somit das Maß an Beeinflussung des Teilchenstrahls durch den Strahleigenschafts-Überwachungsprozess gering gehalten, werden. Insbesondere kann mit der beweglichen Ausgestaltung gewährleistet werden, dass der Strahlpositions-Monitor gegebenenfalls dem gemäß der Rasterscanvorschrift eines Raster-Scan-Therapieverfahrens verschobenen Halobereich des Strahls nachgeführt wird. Darüber hinaus erleichtert die Beweglichkeit der Strahlpositions-Monitore die Bestimmung der Kalibrationsdaten.

In einer weiteren vorteilhaften Ausgestaltung kann die Strahlführungseinheit Scan-Magnete zur Durchführung des Rasterscannens aufweisen, wobei die Kontrolleinheit derart eingerichtet ist, dass erste Stellsignale zur Durchführung eines Raster-Scan-Therapieverfahrens und an Hand der ermittelten Abweichungen zweite Stellsignale zur Anpassung der Strahlposition erzeugt werden, und dass aus den ersten Stellsignalen und den zweiten Stellsignalen resultierende dritte Stellsignale erzeugt und an die Scan-Magnete übermittelt werden. Auf diese Weise können die Scan-Magnete, welche als magnetoptische Elemente für eine durch das Raster-Scan-Therapieverfahren vorgegebene Verschiebung des Strahls der geladenen Teilchen sorgen, entlang des Strahlwegs, vorzugsweise in einem in der Gantry befindlichen Strahlwegabschnitt, gleichzeitig für die Korrektur der unvorhersehbaren Orts- oder Impulsabweichungen, welche beispielsweise bei einer Drehung der Gantry oder bei der Variation des Erregerstroms in den Magnetfeldspulen auftreten, mit verwendet werden. Der vorrichtungstechnische Aufwand zur Umsetzung der Teilchenstrahl-Therapieanlage kann somit vermindert werden.

Alternativ kann die Strahlführungseinheit Scan-Magnete zur Durchführung des Rasterscannens und Korrekturmagnete, insbesondere aus lamelliertem Eisen, aufweisen, wobei die Kontrolleinheit derart eingerichtet ist, dass erste Stellsignale zur Durchführung eines Raster-Scan-Therapieverfahrens und an Hand der ermittelten Abweichungen zweite Stellsignale zur Anpassung der Strahlposition erzeugt werden, und dass die ersten Stellsignale an die Scan-Magnete und die zweiten Stellsignale an die Korrekturmagnete übermittelt werden.

Die Scan-Magnete, gegebenenfalls mit Korrekturfunktion, und/oder die Korrekturmagnete können die Isteigenschaften des Teilchenstrahls in vier Dimensionen des transversalen Phasenraums (zwei räumliche Dimensionen transversal zum Strahlweg und die dazu konjugierten Impulsdimensionen) an die Solleigenschaften anpassen. Um eine schnelle Korrektur durchführen zu können, ist als Werkstoff für die Korrekturmagnete insbesondere lamelliertes Eisen geeignet. Der Einfluss von Wirbelstromeffekten wird dadurch auf einem niedrigen Niveau gehalten, und die zur Führung und Korrektur des Strahls notwendigen Magnetfelder können in Bruchteilen einer Sekunde auf die erforderlichen Werte eingestellt werden.

Ebenso ist es möglich, Scan-Magnete an der Teilchenstrahl-Therapieanlage vorzusehen, welche sowohl für die Durchführung des Raster-Scan-Therapieverfahrens als auch für die Korrektur der Strahlabweichungen verwendet werden, und gleichzeitig spezielle Korrekturmagnete an anderen Orten entlang des Strahlwegs einzusetzen, welche ausschließlich zur Korrektur der Abweichungen Verwendung finden.

Die Strahlführungseinheit weist vor dem mindestens einen Behandlungsort eine, insbesondere drehbare, Gantry auf. In einer speziellen Ausgestaltung können statt eines Behandlungsraumes auch mehrere Behandlungsräume mit jeweils zugeordneten, voneinander abweichenden Gantrys vorgesehen sein, wobei der Strahl geladener Teilchen an Hand seiner Eigenschaften wie Teilchenenergie oder Divergenzverhalten über eine an diese Eigenschaften angepasste magnetoptische Einrichtung an der Gantry in den entsprechenden Behandlungsraum geleitet wird. Die Gantry ist, insbesondere um eine horizontale Achse, um bis zu 360° rotierbar um den Behandlungsort aus möglichst vielen Winkeln bestrahlen zu können. Die Gantry umfasst ferner insbesondere eine daran montierte magnetoptische Einrichtung mit Strahlablenkelementen und/oder Strahlformungselementen, beispielsweise Multipolmagnete (Dipolmagnete, Quadrupolmagnete, Sextupolmagnete, ...) zur Strahlführung und Strahlformung, um den Teilchenstrahl von einer vorherigen Strahlrichtung auf den Behandlungsort hin zu leiten, umzulenken, zu fokussieren und/oder zu kollimieren.

Die Gantry weist mindestens einen Strahlpositions-Monitor auf. Auf diese Weise kann die Bestimmung der Isteigenschaften ortsnah an dem Behandlungsort durchgeführt werden, was eine etwaig erforderliche Korrektur mit erhöhter Genauigkeit ermöglicht.

Die Strahlführungseinheit kann mehrere magnetoptische Einrichtungen, insbesondere in Form von Multipolmagneten, aufweisen.

Vorzugsweise ist dem Strahlpositions-Monitor im Strahlweg ein magnetoptischer Kollimator nachgeordnet. Mittels eines magnetoptischen Kollimators kann insbesondere gezielt ein Ausschnitt des von den Teilchen des Strahls abgebildeten Phasenraumvolumens zur Weiterführung an den Behandlungsort ausgewählt werden.

Gemäß einer weiteren Lehre der vorliegenden Erfindung wird die Aufgabe auch durch ein Teilchenstrahl-Korrekturverfahren nach Anspruch 12 gelöst.

Die Kontrolleinheit kann die Isteigenschaften des Strahls aus den Messungen des Strahlpositions-Monitors unter Einbeziehung von Kalibrationsdaten ermitteln.

Die Kontrolleinheit kann weiterhin erste Stellsignale zur Durchführung eines Raster-Scan-Therapieverfahrens und an Hand der ermittelten Abweichungen zweite Stellsignale zur Anpassung der Strahlposition erzeugen, wobei aus den ersten Stellsignalen und den zweiten Stellsignalen resultierende dritte Stellsignale erzeugt und an Scan-Magnete, welche an der Strahlführungseinheit zur Durchführung des Rasterscannens angeordnet sind, übermittelt werden.

Alternativ kann die Kontrolleinheit erste Stellsignale zur Durchführung eines Raster-Scan-Therapieverfahrens und an Hand der ermittelten Abweichungen zweite Stellsignale zur Anpassung der Strahlposition erzeugen, wobei die ersten Stellsignale an Scan-Magnete, welche an der Strahlführungseinheit zur Durchführung des Rasterscannens angeordnet sind, und die zweiten Stellsignale an Korrekturmagnete, insbesondere aus lamelliertem Eisen, welche an der Strahlführungseinheit angeordnet sind, übermittelt werden.

Hinsichtlich weiterer Vorteile oder vorteilhafter Ausführungsformen des Verfahrens wird auf die Ausführungen zu der erfindungsgemäßen Teilchenstrahl-Therapieanlage verwiesen. Bevorzugt ist die Verwendung einer wie vorstehend beschriebenen Teilchenstrahl-Therapieanlage zur Bestrahlung von, insbesondere menschlichem, Gewebe.

Besonders bevorzugt ist die Verwendung einer wie vorstehend beschriebenen Teilchenstrahl-Therapieanlage in der Tumortherapie, insbesondere am Menschen.

Es gibt vielfältige Möglichkeiten, die erfindungsgemäße Teilchenstrahl-Therapieanlage oder das erfindungsgemäße Verfahren auszugestalten und weiterzubilden. Hierzu wird einerseits auf die abhängigen Patentansprüche, andererseits auf die Erläuterung von Ausführungsbeispielen in Verbindung mit der beigefügten Zeichnung verwiesen. In der Zeichnung zeigen:
- Fig. 1: eine schematische Übersichtsdarstellung einer Teilchenstrahl-Therapieanlage,
- Fig. 2a-b: den Aufbau von Strahlführungseinheiten an einer Gantry,
- Fig. 3a-c: mehrere Ausführungsbeispiele für Strahleigenschafts-Monitore, und
- Fig. 4: Ergebnisse einer Monte-Carlo-Simulation zur Bestimmung der Genauigkeit der Ortsabweichung.

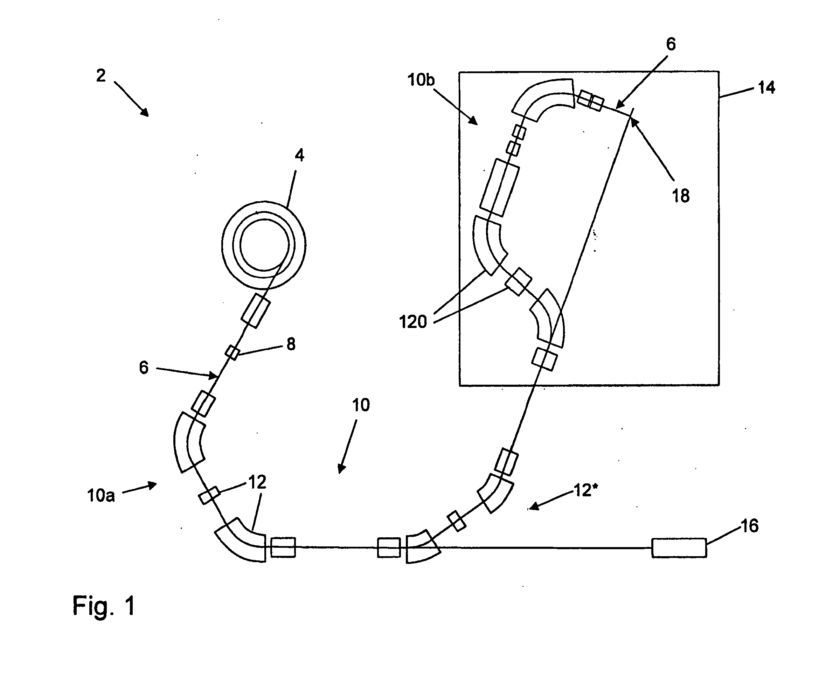

Figur 1 zeigt in einer schematischen Übersichtsdarstellung ein Ausführungsbeispiel einer Teilchenstrahl-Therapieanlage 2 gemäß der vorliegenden Erfindung. Die Teilchenstrahl-Therapieanlage 2 weist eine Beschleunigereinrichtung 4 in Form eines, insbesondere supraleitenden, Zyklotrons auf, welches die geladenen Teilchen bis zu einer Energie von 200 bis 250 MeV beschleunigen und in Pulsen mit einer Frequenz von beispielsweise etwa 72 MHz bereitstellen kann. Zur Korrektur der Energie des Teilchenstrahls, sofern nicht die Maximalenergie erforderlich ist, befindet sich im Strahlweg 6 eine Energiekorrektureinheit 8 in Form eines Degraders. Nach Durchlaufen des Degraders 8 verläuft der Strahlweg 6 des Teilchenstrahls durch eine Strahlführungseinheit 10. Die Strahlführungseinheit 10 umfasst in diesem Beispiel eine Strahltransfereinheit 10a mit mehreren magnetoptischen Elementen 12, welche den Teilchenstrahl ausgehend von der Beschleunigereinrichtung 4 einem Behandlungsraum 14 zuführen.

Mittels einer magnetoptischen Weiche 12*, beispielsweise in Form eines kleinen Systems von Dipol- und Quadrupolmagneten, wird der Teilchenstrahl in Richtung auf den Behandlungsraum 14 umgelenkt. Hinter der magnetoptischen Weiche 12* befindet sich in der Strahlverlängerung aus Sicherheitsgründen eine Strahlauffangeinheit 16.

In dem Behandlungsraum 14 sind magnetoptische Elemente 120 in Form von Strahlablenkelementen und Strahlformungselementen (zum Beispiel zur Kollimation oder Fokussierung) angeordnet, welche als 10b ebenfalls der Strahlführungseinheit 10 zugeordnet sind. Die magnetoptischen Elemente 120 sorgen dafür, dass nach vorgegebenen Bedingungen, beispielsweise hohem Fokussierungsgrad und/oder hohem Transmissionsgrad, der Teilchenstrahl dem Behandlungsort 18 zugeführt wird. In dem Behandlungsraum 14 ist der entsprechende Abschnitt 10b der Strahlführungseinheit 10 an einer rotierbaren Gantry (nicht gezeigt) angeordnet, mittels derer die den Strahlweg 6 steuernden magnetoptischen Elemente 120 um den Behandlungsort 18 rotiert werden können (bis zu 360°), um unterschiedliche Bestrahlungswinkel einzustellen.

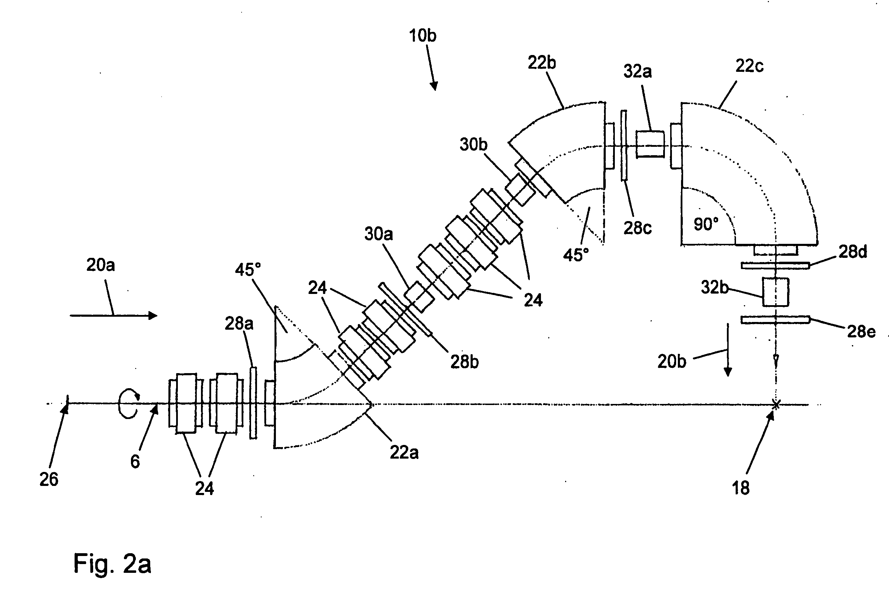

Ein möglicher Aufbau der Strahlführungseinheit 10b an einer Gantry ist exemplarisch in Fig. 2a dargestellt. Aus der von der magnetoptischen Weiche 12* eingestellten Strahleinfallsrichtung 20a wird der Teilchenstrahl im weiteren Strahlwegverlauf dreimal umgelenkt, um schließlich eine Richtung 20b anzunehmen, welche senkrecht zur ursprünglichen Strahleinfallsrichtung 20a liegt. Der Schnittpunkt der verlängerten Strahleinfallsrichtung 20a mit dem dreimal umgelenkten Strahl wird als Isozentrum bezeichnet und kennzeichnet den Behandlungsort 18, also den Ort, an dem ein Target dem Teilchenstrahl ausgesetzt wird. Die Strahlführungseinheit 10b als Transportsystem des Teilchenstrahls kann ein Gewicht von mehreren hundert Tonnen aufweisen und ist dementsprechend auf einem hochstabilen Traggestell ("Gantry" ₋ nicht dargestellt) montiert, welches um bis zu 360° in diesem Beispiel um die Strahleinfallsrichtung 20a als Drehachse bewegt werden kann.

Durch die der Strahlführungseinheit 10b an der Gantry zugeordneten magnetoptischen Elemente, in diesem Beispiel drei Ablenkmagnete 22a, 22b, 22c und mehrere Quadrupolmagnete 24, wird der Transport eines möglichst großen Phasenraumvolumens der geladenen Teilchen vom Eintrittspunkt 26 bis zum Isozentrum 18, wo der Strahl unter einem Winkel nahe 90° gegenüber der Rotationsachse eintrifft, sichergestellt. Um die erforderliche Rotationssymmetrie zu gewährleisten, weist der Teilchenstrahl am Eintrittspunkt 26 der Gantry vorzugsweise selbst eine Rotationssymmetrie in der zum Strahlweg 6 transversalen Raumkoordinaten-Ebene und eine Impulsdispersion von nahezu Null auf. Auf diese Weise kann bei der Anwendung eines Raster-Scan-Therapieverfahrens ein kleiner Strahlquerschnitt von nur wenigen Quadratmillimetern am Isozentrum 18 erreicht werden.

Der Teilchenstrahl verläuft in der Gantry zunächst entlang der durch den Eintrittspunkt 26 und das Isozentrum 18 verlaufenden Rotationsachse der Gantry. Er tritt sodann in die ersten beiden Quadrupolmagnete 24 ein, in welchen er refokussiert wird. Sodann durchläuft er einen ersten Strahleigenschafts-Monitor 28a, welcher in diesem Beispiel als Anordnung von Straw Tubes ausgebildet ist, und anschließend einen als Umlenkmagneten 22a fungierenden Dipolmagneten, welcher den Teilchenstrahl um 45° ablenkt. Nach Durchlaufen zweier weiterer Quadrupolmagneten 24 passiert er einen weiteren Strahleigenschafts-Monitor 28b in Form einer Anordnung von Straw Tubes und wird in dem diesem nachgeordneten Korrekturmagneten 30a bezüglich einer zum Strahlweg 6 transversalen Raumkoordinate (vorzugsweise ebenso der entsprechenden Impulskoordinate) gegebenenfalls korrigiert. Nach Passieren der Quadrupolmagneten 24 durchläuft er einen zweiten Korrekturmagneten 30b, wo er bezüglich der zu der vorstehend genannten Raumkoordinate orthogonalen Raumkoordinate (vorzugsweise ebenso bezüglich der entsprechenden Impulskoordinate) gegebenenfalls korrigiert wird. Im Folgenden passiert er einen weiteren 45°-Umlenkmagneten 22b, so dass er wieder parallel zur Rotationsachse der Gantry propagiert. Im Folgenden durchläuft er einen weiteren Strahleigenschafts-Monitor 28c in Form einer Anordnung von Straw Tubes und daran anschließend einen ersten Scan-Magneten 32a zur Durchführung eines Rasterscannens, durch welchen der Strahl über den gewünschten Orts- und Impulsbereich im Target gerastert wird. Schließlich wird er durch einen weiteren Umlenkmagneten 22c um 90° in Richtung des im Isozentrum 18 befindlichen Targets (beispielsweise das Tumorgewebe) abgelenkt und mittels eines weiteren Scan-Magneten 32b über den gewünschten Orts- und Impulsbereich im Target gerastert, so dass dieses in seiner vollständigen Ausdehnung erfasst wird. Dies wird über zwei weitere im Strahlweg 6 dem zweiten Scan-Magneten 32b vor- und nachgeordnete Strahleigenschafts-Monitore 28d, 28e in Form von Straw-Tube-Anordnungen kontrolliert.

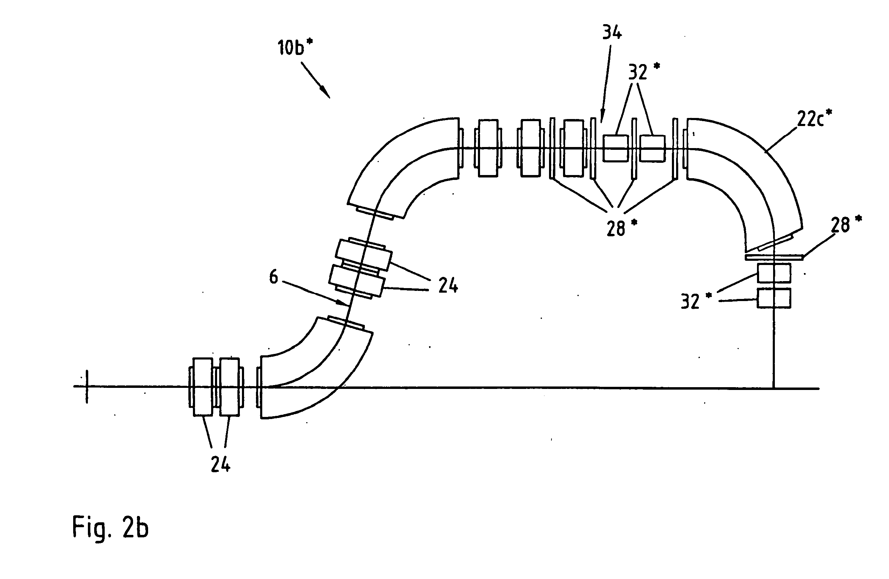

Ein ähnlicher Aufbau der Strahlführungseinheit 10b* an einer Gantry ist auch in Figur 2b gezeigt. Funktionell vergleichbare Elemente sind darin mit den gleichen Bezugszeichen wie in Figur 2a versehen. Im Folgenden werden lediglich die Unterschiede zu der Strahlführungseinheit 10b in Figur 2a erläutert. Im Unterschied zu Figur 2a weist die Strahlführungseinheit 10b* vier Scan-Magnete 32* auf, von denen jeder zum Rasterscannen des Teilchenstrahls in einer zum Strahlweg 6 transversalen Dimension verwendet werden kann. Der Teilchenstrahl kann demnach durch die Scan-Magnete 32* nach Vorgabe des Raster-Scan-Therapieverfahrens gezielt in seiner räumlichen Ausrichtung verändert werden. Zusätzlich zur Ausführung des Raster-Scan-Therapieverfahrens können die Scan-Magnete 32* jedoch auch die Korrekturen der unvorhersehbaren Strahlabweichungen ausführen. Auf diese Weise kann auf die Anordnung zusätzlicher, speziell auf die Korrektur der Abweichungen ausgelegter Korrekturmagnete (siehe 30a, 30b in Figur 2a) verzichtet werden. Damit lässt sich der vorrichtungstechnische Aufwand der Teilchenstrahl-Therapieanlage verringern. An der mit 34 bezeichneten Stelle im Strahlweg 6 kann dem entsprechenden Strahleigenschafts-Monitor 28* ein Kollimator (nicht dargestellt) nachgeordnet sein. Durch diese Variante der Strahlführungseinheit 10b* an der Gantry kann die Breite des Strahlprofils verringert werden. Die im Strahlweg 6 folgenden strahlführenden, magnetoptischen Elemente, insbesondere der 90°-Umlenkmagnet 22c*, lassen sich somit vorrichtungstechnisch vereinfachen. Durch diese vereinfachte Auslegung der magnetoptischen Elemente wird eine vorteilhafte Kosteneinsparung erzielt. Besonders geeignet ist diese Variante, wenn die beiden der Stelle 34 im Strahlweg 6 vorgelagerten Strahleigenschafts-Monitore 28* für eine Korrektur der Strahleigenschaftsabweichungen genügen.

Die Messdaten von in diesem Beispiel fünf Strahleigenschafts-Monitoren 28* werden von einer Kontrolleinheit (nicht gezeigt) unter Heranziehung von Kalibrationsdaten dazu verwendet, Abweichungen der Isteigenschaften des Strahls von den Solleigenschaften zu bestimmen und an Hand dieser Abweichungen Stellsignale zur Korrektur zu erzeugen. Diese zweiten Stellsignale zur Korrektur können mit den ersten Stellsignalen zur Durchführung des Raster-Scan-Therapieverfahrens zu resultierenden dritten Stellsignalen verarbeitet, gewissermaßen einander überlagert, werden.

Zur Registrierung und Verarbeitung der, gegebenenfalls analogen, Signale von mindestens zwei Strahleigenschafts-Monitoren 28, 28* (wie in den Figuren 2a und 2b gezeigt) und deren digitaler Aufbereitung wird eine schnelle in der Kontrolleinheit implementierte Kontrollelektronik verwendet. Hierbei können die Signale aller an der Peripherie des Strahls befindlichen Strahleigenschafts-Monitore 28, 28*, sowie gegebenenfalls weiterer im Strahlweg 6 vorhandener Monitore, für die Berechnung der Orts- und Impulsabweichung des Strahls und die Berechnung der zur Korrektur des Strahls notwendigen Stellsignale für die Korrekturmagnete (siehe 30a, 30b in Figur 2a) einbezogen werden. Damit ist eine automatisch regelnde Korrektur des Teilchenstrahls längs des gesamten Strahlwegs 6, insbesondere im Bereich der Gantry, möglich.

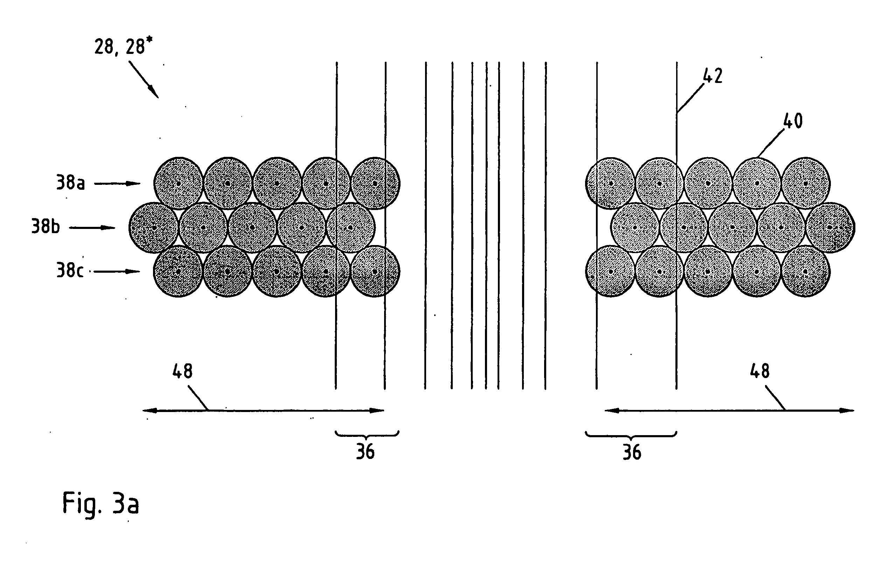

Figur 3a zeigt das Prinzip eines Strahleigenschafts-Monitors 28, 28*, welcher eine zum Strahlweg 6 orthogonale Detektionsfläche aufweist, wobei die Detektionsfläche im Halobereich 36 mit dem Strahlquerschnitt überlappt, welcher also wenigstens teilweise in den Halobereich 36 des Teilchenstrahls hineinreicht. Der Strahleigenschafts-Monitor 28, 28* weist in diesem Beispiel - in Richtung des Strahlwegs 6 hintereinander - drei Lagen 38a, 38b, 38c zylindrischer Ionisationskammern 40 - Straw Tubes - auf, wobei jeweils drei Lagen 38a, 38b, 38c auf unterschiedlichen Seiten des Strahls einander diametral gegenüberliegen. Es können auch zwei oder mehr Lagen der zylindrischen Ionisationskammern 40 vorgesehen werden. Die zylindrischen Ionisationskammern 40 lassen sich dabei im Vakuum der Strahlführungseinheit 10b* betreiben. Sie sind mittels einer Stelleinrichtung (nicht dargestellt), gegebenenfalls unabhängig, von beiden Seiten her in Richtung der Sollposition des hier durch geringer beabstandete Strahllinien 42 angedeuteten Strahlzentrums, insbesondere in einer Richtung transversal zum Strahlweg 6, verschiebbar.

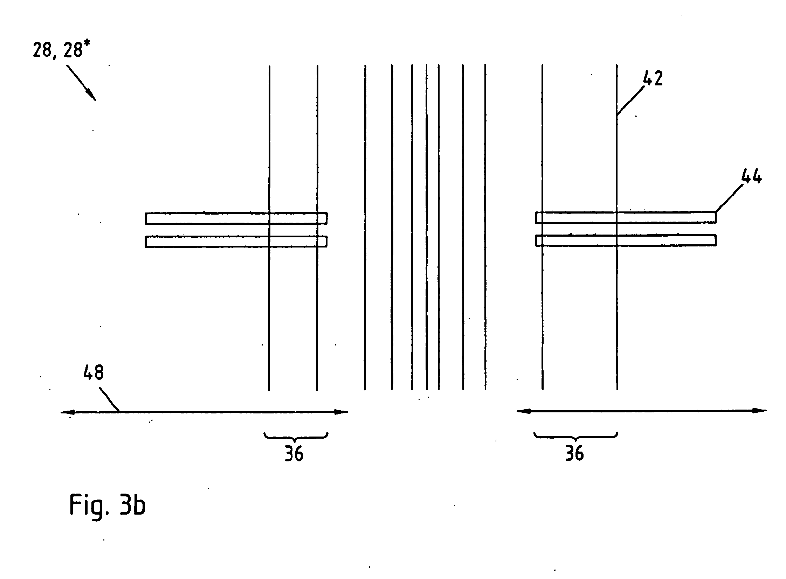

Figur 3b zeigt das Prinzip eines weiteren Strahleigenschafts-Monitors 28, 28*, welcher auf zwei Seiten des Teilchenstrahls einander diametral gegenüberliegend zwei dünne Szintillationszähler 44 aufweist. Diese Szintillationszähler 44 können ebenfalls im Vakuum betrieben werden. Die auf einer Seite entlang des Strahlwegs 6 hintereinander angeordneten Szintillationszähler 44 werden vorzugsweise in Koinzidenz geschaltet. Das beim Durchgang eines Teilchens aus dem Halobereich 36 des Strahls in dem Szintillatormaterial erzeugte Licht wird über Lichtleiter (gegebenenfalls mit Wellenlängenschieber) auf die Fotokathode eines Fotomultipliers geführt (nicht gezeigt). Durch Koinzidenzschaltung der Strompulse der Fotomultiplier kann sichergestellt werden, dass auf jeder Seite des Strahls nur Signale von durchlaufenden geladenen Teilchen registriert werden. Die Ausdehnung des Szintillationsmaterials in Richtung des Strahlwegs beträgt vorzugsweise 0,5 bis 2 mm.

Wie an Hand der durch die Abstände der einzelnen Strahllinien 42 angedeuteten Leistungsdichte zu erkennen ist, muss das Profil des Strahls geladener Teilchen nicht notwendigerweise symmetrisch sein.

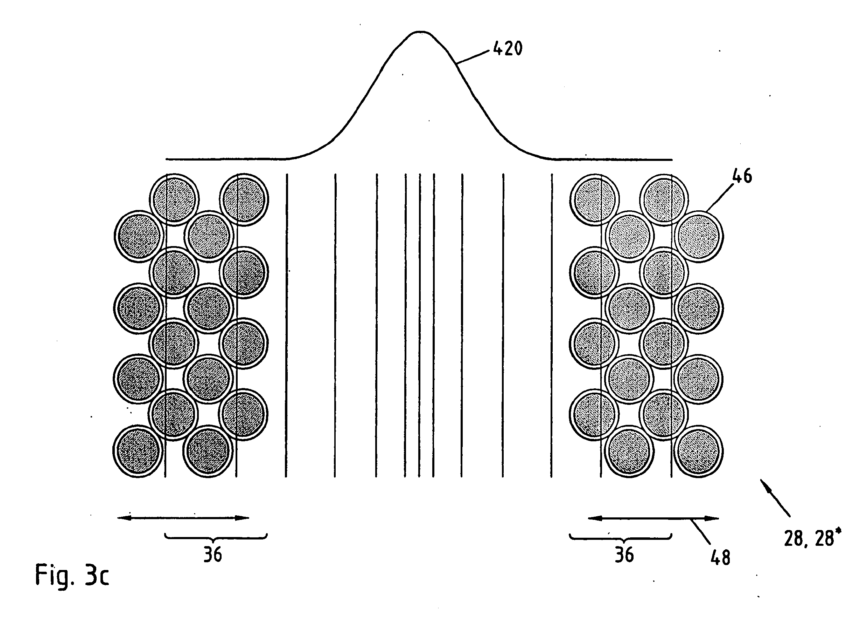

Figur 3c zeigt das Prinzip eines weiteren Strahleigenschafts-Monitors 28, 28*, welcher mehrere Lagen organischer oder anorganischer szintillierender Fasern 46 aufweist. Auch diese Fasern 46 sind vakuumtauglich. Die Auslesung der erfassten Signale erfolgt entweder direkt oder über einen zwischengeschalteten Lichtleiter und Vielkanal-Fotomultiplier (nicht dargestellt). Zur Verdeutlichung ist ein durch die Strahllinien 42 angedeuteter, möglicher Intensitätsverlauf des Strahls geladener Teilchen in Form einer Normalverteilung 420 in Figur 3c abgebildet.

Organisches Szintillatormaterial ist nur begrenzt strahlenhart. Um die Lebensdauer von Strahleigenschafts-Monitoren 28, 28*, welche organisches Szintillatormaterial enthalten, zu verlängern, kann vorgesehen werden, den Szintillationszähler nach einer gewissen Betriebsdauer so zu verschieben, dass ein bisher für die Erfassung der Strahleigenschaften verwendeter Bereich des Szintillatormaterials aus dem Halobereich 36 heraus und ein bislang nicht für die Erfassung verwendeter Bereich des Szintillatormaterials in den Halobereich 36 hinein bewegt wird.

Wie durch die doppelköpfigen Pfeile 48 in den Figuren 3a bis 3c angedeutet, können die Strahleigenschafts-Monitore 28, 28* von der Peripherie des Strahls her symmetrisch oder unsymmetrisch in Richtung der Sollposition des Zentrums des Strahls bewegt werden.

Zusätzlich zu Strahleigenschafts-Monitoren 28, 28*, welche unmittelbar mit Teilchen des Strahls im Halobereich 36 wechselwirken, können auch Strahleigenschafts-Monitore (nicht dargestellt) verwendet werden, welche außerhalb des Strahls an dessen Peripherie angeordnet sind und die Strahleigenschaften an Hand der von den fließenden Teilchen des Strahls erzeugten elektromagnetischen Felder erfassen. Der Strahleigenschafts-Monitor 28, 28* kann beispielsweise wenigstens eine außerhalb des Strahls angeordnete Elektrode (nicht gezeigt), in welcher elektrische Ladungen influenzierbar sind, aufweisen. Der Strahleigenschafts-Monitor 28, 28* kann zusätzlich wenigstens einen SQUID-Detektor (nicht gezeigt) aufweisen.

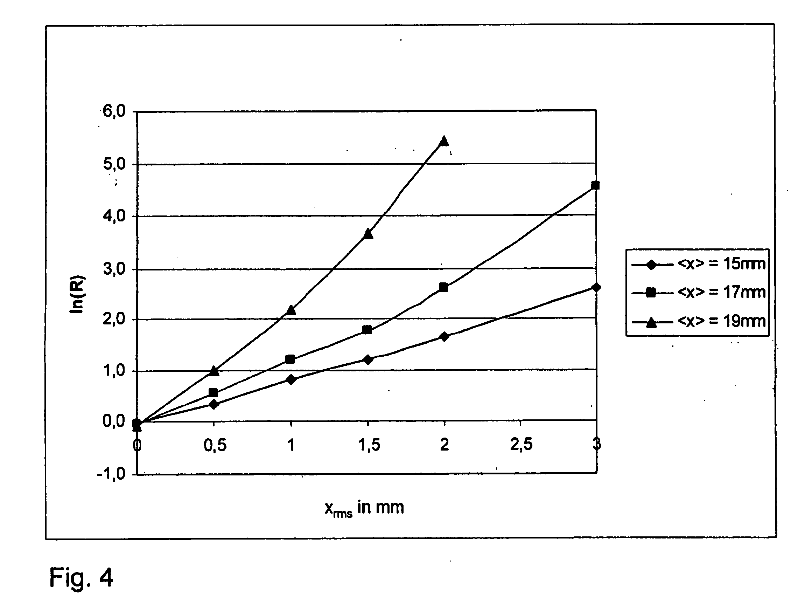

Figur 4 gibt ein Beispiel für die Genauigkeit, mit welcher eine Ortsabweichung des Strahl-Schwerpunkts von der Sollposition bestimmt werden kann. In diesem Beispiel wird angenommen, dass sich der Strahleigenschafts-Monitor 28, 28* in der Gantry unmittelbar vor den Scan-Magneten (siehe Figur 2b) befindet.

Es werden die Teilchen-Zählraten N1 und N2 in zwei einander diametral gegenüberliegenden Bereichen in der Halo des Strahls erfasst. Es wird ferner ein zweidimensionales Raumkoordinatensystem aufgestellt, welches eine Ebene senkrecht zum Strahlweg 6 aufspannt. Die beiden transversalen Raumachsen sind orthogonal zueinander. Der Nullpunkt der beiden Raumkoordinatenachsen liegt dabei im Schwerpunkt des Strahls (also dem Ort mit der höchsten Teilchenzahl bzw. höchsten Leistungsdichte), wenn sich dieser in der Sollposition befindet. Der Variablen N1 wird die Zählrate im Bereich positiver Raumkoordinaten entlang einer Achse (also einem ersten Strahleigenschafts-Monitor 28, 28*) und der Variablen N2 die Zählrate im Bereich negativer Raumkoordinaten entlang dieser Achse (also einem dem ersten Strahleigenschafts-Monitor 28, 28* diametral gegenüberliegenden zweiten Strahleigenschafts-Monitor 28, 28*) zugewiesen.

Aufgetragen ist die Größe ln(R)=ln(N1/N2) als Funktion der Abweichung xᵣₘₛ (rms: root mean square, mittlere quadratische Abweichung) der Istposition von der Sollposition in Millimetern am Ort der Strahleigenschafts-Monitore 28, 28* entlang des Strahlwegs 6. Dabei wird vorausgesetzt, dass die Strahleigenschafts-Monitore 28, 28* symmetrisch zur Sollposition, also dem Nullpunkt, angeordnet sind. Es wird weiterhin angenommen, dass N1 größer oder wenigstens gleich N2 ist - d.h. der Strahl wird in Richtung auf den Strahleigenschafts-Monitor 28, 28*, welchem die Zählrate N1 zugeordnet ist, aus seiner Sollposition verschoben. Die Größe <x> rechts in Figur 4 bezeichnet den mittleren Abstand der beiden Strahleigenschafts-Monitore 28, 28* vom Nullpunkt. Die Kurven sind mittels detaillierter Monte-Carlo-Simulationen ermittelt.

Der Fehler der Messung des logarithmischen Verhältnisses der zählraten ist deutlich kleiner als ein Prozent, die daraus resultierende Genauigkeit der Bestimmung von xᵣₘₛ liegt folglich im Bereich weniger Zehntel Millimeter. Dies ist ausreichend, um eine praktikable Abweichungskorrektur durchzuführen.

Die physikalischen Grundlagen der Wechselwirkung von Protonen, mit Materie und die zur Berechnung der Eigenschaften von Strahlführungssystemen verwendeten Programme sind in den folgend genannten wissenschaftlichen Berichten beschrieben:
- Particle Data Group, W.-M. Yao et al., "The Review of Particle Physics", Journal of Physics G33 (2006)1 and update 2008.
- Karl L. Brown, Sam K. Howry, "TRANSPORT, A Computer Program for Designing Charged Particle Beam Transport Systems", SLAC Report No. 91 (1970) and later updates of the TRANSPORT program by U. Rohrer and others.
- U. Rohrer, "PSI Graphic TURTLE Framework based on a CERN-SLAC-FERMILAB version by K.L. Brown et al.", http://people.web.psi.ch/rohrer-u/turtle.htm (2008).
- J. Drees, "Passage of Protons through Thick Degraders", Cryoelectra Report Sept. 2008.

## Patentansprüche

1. Teilchenstrahl-Therapieanlage (2)
mit einer Beschleunigereinrichtung (4) zur Bereitstellung eines Strahls geladener Teilchen,
mit einer Strahlführungseinheit (10, 10a, 10b; 10b*) zur Führung des Strahls ausgehend von der Beschleunigereinrichtung (4) zu einem Behandlungsort (18), wobei die Strahlführungseinheit (10, 10a, 10b; 10b*) eine rotierbare Gantry mit wenigstens einem entlang des Strahlwegs (6) in der Gantry angeordneten Strahlpositions-Monitor (28; 28*) zur Erfassung der transversalen Strahlposition und/oder des zum Strahlweg (6) transversalen Impulses aufweist, und
mit einer mit dem Strahlpositions-Monitor (28; 28*) und der Strahlführungseinheit (10, 10a, 10b; 10b*) kommunizierenden Kontrolleinheit zur Ermittlung von Abweichungen der Istposition von der Sollposition des Strahls und zur Übermittlung von Stellsignalen zur Anpassung der Strahlposition an die Strahlführungseinheit (10, 10a, 10b; 10b*) an Hand der Abweichungen, wobei der Strahlpositions-Monitor (28; 28*) derart an der zum Strahlweg (6) senkrechten Peripherie des Strahls angeordnet ist, dass die transversale Strahlposition und/oder der zum Strahlweg (6) transversale Impuls durch unmittelbare Wechselwirkung mit Teilchen des Strahls im Halobereich (36) in wenigstens einer Dimension erfasst wird.

2. Teilchenstrahl-Therapieanlage nach Anspruch 1,
**dadurch gekennzeichnet, dass**
der Strahlpositions-Monitor (28; 28*) eine zum Strahlweg (6) orthogonale Detektionsfläche aufweist, die im Halobereich (36) mit dem Strahlquerschnitt überlappt.

3. Teilchenstrahl-Therapieanlage nach Anspruch 1 oder 2,
**dadurch gekennzeichnet, dass**
die Kontrolleinheit derart eingerichtet ist, dass die Ist-Position des Strahls aus den Messungen des Strahlpositions-Monitors (28; 28*) unter Einbeziehung von Kalibrationsdaten ermittelt wird.

4. Teilchenstrahl-Therapieanlage nach einem der Ansprüche 1 bis 3,
**dadurch gekennzeichnet, dass**
zwei Strahlpositions-Monitore (28; 28*) einander diametral gegenüberliegend an der Peripherie des Strahls angeordnet sind.

5. Teilchenstrahl-Therapieanlage nach einem der Ansprüche 1 bis 4,
**dadurch gekennzeichnet, dass**
der Strahlpositions-Monitor (28; 28*) wenigstens einen Szintillationszähler (40; 46) aufweist.

6. Teilchenstrahl-Therapieanlage nach Anspruch 5,
**dadurch gekennzeichnet, dass**
das Szintillationsmaterial in Richtung des Strahlwegs (6) eine Dicke von etwa 0,5 bis 2 mm aufweist.

7. Teilchenstrahl-Therapieanlage nach einem der Ansprüche 1 bis 6,
**dadurch gekennzeichnet, dass**
der Strahlpositions-Monitor (28; 28*) wenigstens eine, vorzugsweise zylindrische, Ionisationskammer (44) aufweist.

8. Teilchenstrahl-Therapieanlage nach einem der Ansprüche 1 bis 7,
**dadurch gekennzeichnet, dass**
der Strahlpositions-Monitor (28; 28*) transversal zum Strahlweg (6) beweglich ist.

9. Teilchenstrahl-Therapieanlage nach einem der Ansprüche 1 bis 8,
**dadurch gekennzeichnet, dass**
die Strahlführungseinheit (10, 10a, 10b; 10b*) Scanmagnete mit korrekturfunktion (32, 32*) zur Durchführung des Rasterscannens aufweist, wobei die Kontrolleinheit derart eingerichtet ist, dass erste Stellsignale zur Durchführung eines Raster-Scan-Therapieverfahrens und an Hand der ermittelten Abweichungen zweite Stellsignale zur Anpassung der Strahlposition erzeugt werden, und dass aus den ersten Stellsignalen und den zweiten Stellsignalen resultierende dritte Stellsignale erzeugt und an die Scan magnete mit korrekturfunktion (32, 32*) zur Durchführung des korrigierten Raster-Scan-verfahrens übermittelt werden.

10. Teilchenstrahl-Therapieanlage nach einem der Ansprüche 1 bis 9,
**dadurch gekennzeichnet, dass**
die Strahlführungseinheit (10, 10a, 10b; 10b*) Scan-Magnete (32, 32*) zur Durchführung des Rasterscannens und Korrekturmagnete (30a, 30b), insbesondere aus lamelliertem Eisen, aufweist, wobei die Kontrolleinheit derart eingerichtet ist, dass erste Stellsignale zur Durchführung eines Raster-Scan-Therapieverfahrens und an Hand der ermittelten Abweichungen zweite Stellsignale zur Anpassung der Strahlposition erzeugt werden, und dass die ersten Stellsignale an die Scan-Magnete (32; 32*) und die zweiten Stellsignale an die Korrekturmagnete (30a, 30b) übermittelt werden.

11. Teilchenstrahl-Therapieanlage nach einem der Ansprüche 1 bis 11,
dadurch gekenntzeichnet, **dass**
dem unmittelbar mit den geladenen Teilchen des Strahls im Halobereich (36) wechselwirkenden Strahlpositions-Monitor (28; 28*) im Strahlweg (6) ein magnetoptischer Kollimator nachgeordnet ist.

12. Verfahren, umfassend:
- Ermitteln von Abweichungen der Ist-Position von der SollPosition eines durch eine Beschleunigereinrichtung (4) einer Teilchenstrahl-Therapieanlage (2) bereitgestellten und mittels einer Strahlführungseinheit (10, 10a, 10b; 10b*) der Teilchenstrahl-Therapieanlage (2) von der Beschleunigereinrichtung (4) zu einem Behandlungsort (18) geführten Strahls geladener Teilchen basierend auf der durch wenigstens einen entlang des Strahlwegs (6) in einer rotierbaren Gantry der Strahlführungseinheit (10, 10a, 10b; 10b*) angeordneten Strahlpositions-Monitor (28; 28*) zur Erfassung der transversalen Strahlposition und/oder des zum Strahlweg (6) transversalen Impulses erfassten transversalen Strahlposition, wobei der Strahlpositions-Monitor (28; 28*) derart an der zum Strahlweg (6) senkrechten Peripherie des Strahls angeordnet ist, dass die transversale Strahlposition und/oder der zum Strahlweg (6) transversale Impuls durch unmittelbare Wechselwirkung mit Teilchen des Strahls im Halobereich (36) in wenigstens einer Dimension erfasst wird, und
- Übermitteln von Stellsignalen zur Anpassung der Stahlposition an die Strahlführungseinheit (10, 10a, 10b; 10b*) an Hand der Abweichungen,
wobei das Ermitteln der Abweichungen und das Übermitteln der Stellsignale durch eine mit dem Strahlpositions-Monitor (28; 28*) und der Strahlführungseinheit (10, 10a, 10b; 10b*) kommunizierende Kontrolleinheit der Teilchenstrahl-Therapieanlage erfolgt.

13. Verfahren nach Anspruch 12,
**dadurch gekennzeichnet, dass**
die Ist-Position des Strahls aus den Messungen des Strahlpositions-Monitors (28; 28*) unter Einbeziehung von Kalibrationsdaten ermittelt wird.

14. Verfahren nach einem der Ansprüche 12 bis 13,
**dadurch gekennzeichnet, dass**
die Strahlführungseinheit (10, 10a, 10b; 10b*) Scanmagnete mit korrekturfunktion (32, 32*) zur Durchführung des Rasterscannens aufweist, wobei die Kontrolleinheit derart eingerichtet ist, dass erste Stellsignale zur Durchführung eines Raster-Scan-Therapieverfahrens und an Hand der ermittelten Abweichungen zweite Stellsignale zur Anpassung der Strahlposition erzeugt werden, und dass aus den ersten Stellsignalen und den zweiten Stellsignalen resultierende dritte Stellsignale erzeugt und an die Scan magnete mit korrekturfunktion (32, 32*) zur Durchführung des korrigierten Raster-Scan-verfahrens übermittelt werden.

15. Verfahren nach einem der Ansprüche 12 bis 13,
**dadurch gekennzeichnet, dass**
die Strahlführungseinheit (10, 10a, 10b; 10b*) Scan-Magnete (32, 32*) zur Durchführung des Rasterscannens und Korrekturmagnete (30a, 30b), insbesondere aus lamelliertem Eisen, aufweist, wobei die Kontrolleinheit derart eingerichtet ist, dass erste Stellsignale zur Durchführung eines Raster-Scan-Therapieverfahrens und an Hand der ermittelten Abweichungen zweite Stellsignale zur Anpassung der Strahlposition erzeugt werden, und dass die ersten Stellsignale an die Scan-Magnete (32; 32*) und die zweiten Stellsignale an die Korrekturmagnete (30a, 30b) übermittelt werden.

## Claims

1. A particle-beam therapy system (2)
with acceleration equipment (4) for providing a beam of charged particles,
with a beam-guide unit (10, 10a, 10b; 10b*) for guiding the beam emanating from the acceleration equipment (4) to a treatment site (18), in which the beam-guide unit (10, 10a, 10b; 10b*) exhibits a rotatable gantry with at least one beam-position monitor (28; 28*) disposed on the gantry along the beam path (6) to determine the transverse beam position and/or the pulse transverse to the beam path (6), and
with a control unit communicating with the beam-position monitor (28; 28*) and the beam-guide unit (10, 10a, 10b; 10b*) to determine deviations of the actual position from the nominal position of the beam and for transmitting position signals to adjust the beam position at the beam-guide unit (10, 10a, 10b; 10b*), with the aid of the deviations, in which the beam-position monitor (28; 28*) is disposed on the periphery of the beam perpendicular to the beam path (6) such that the transverse beam position and/or the pulse transverse to the beam path (6) is detected by direct interaction with particles of the beam in the halo region (36) in at least one dimension.

2. The particle-beam therapy system according to claim 1,
**characterized in that**
the beam-position monitor (28; 28*) exhibits a detection surface orthogonal to the beam path (6), which overlaps with the beam cross-section in the halo region (36).

3. The particle-beam therapy system according to claim 1 or 2,
**characterized in that**
the control unit is proposed such that the actual position of the beam is determined from the measurements of the beam-position monitor (28; 28*) when including calibration data.

4. The particle-beam therapy system according to one of claims 1 through 3,
**characterized in that**
two beam-position monitors (28; 28*) are disposed diametrally opposite one another on the periphery of the beam.

5. The particle-beam therapy system according to one of claims 1 through 4,
**characterized in that**
the beam-position monitor (28; 28*) exhibits at least one scintillation counter (40; 46).

6. The particle-beam therapy system according to claim 5,
**characterized in that**
the scintillation material exhibits a thickness of about 0.5 to 2 mm in the direction of the beam path (6).

7. The particle-beam therapy system according to one of claims 1 through 6, **characterized in that**
the beam-position monitor (28; 28*) exhibits at least one, preferably cylindrical, ionization chamber (44).

8. The particle-beam therapy system according to one of claims 1 through 7, **characterized in that**
the beam-position monitor (28; 28*) is movable transverse to the beam path (6).

9. The particle-beam therapy system according to one of claims 1 through 8, **characterized in that**
the beam-guide unit (10, 10a, 10b; 10b*) exhibits scanning magnets with a correction function (32, 32*) to execute raster scans, in which the control unit is proposed such that first position signals are produced for executing a raster-scan therapy method and, with the aid of the deviations determined, second position signals are produced for adjusting the beam position, and that from the first position signals and the second position signals are produced resultant third position signals and they are transmitted to the scanning magnets with correction function (32, 32*) to execute the corrected raster-scan method.

10. The particle-beam therapy system according to one of claims 1 through 9, **characterized in that**
the beam-guide unit (10, 10a, 10b; 10b*) exhibits scanning magnets (32, 32*) for executing the raster scans and correction magnets (30a, 30b), in particular made of lamellar iron, in which the control unit is proposed such that first position signals are produced for executing a raster-scan therapy method and, with the aid of the deviations determined, second position signals are produced for adjusting the beam position, and that the first position signals are transmitted to the scanning magnets (32; 32*) and the second position signals to the correction magnets (30a, 30b).

11. The particle-beam therapy system according to one of claims 1 through 11, **characterized in that**
a magneto-optical collimator is inserted after the beam-position monitor (28; 28*) which interacts directly with the charged particles of the beam in the halo region (36) in the beam path (6).

12. A method, including:
- determining deviations in the actual position from the nominal position of a beam of charged particles provided by means of acceleration equipment (4) of a particle-beam therapy system (2) and guided by means of a beam-guide unit (10, 10a, 10b; 10b*) of the particle-beam therapy system (2) from the acceleration equipment (4) to a treatment site (18), based on the transverse beam position determined by at least one beam-position monitor (28; 28*) disposed along the beam path (6) on a rotatable gantry of the beam-guide unit (10, 10a, 10b; 10b*) for determining the transverse beam position and/or the pulse transverse to the beam path (6), in which the beam-position monitor (28; 28*) is disposed on the periphery of the beam perpendicular to the beam path (6) such that the transverse beam position and/or the pulse transverse to the beam path (6) is detected by direct interaction with particles of the beam in the halo region (36) in at least one dimension, and
- transmitting the position signals to the beam-guide unit (10, 10a, 10b; 10b*) to adjust the beam position, with the aid of the deviations,
in which the determination of the deviations and the transmission of the position signals occurs by means of a
control unit of the particle-beam therapy system communicating with the beam-position monitor (28; 28*) and the beam-guide unit (10, 10a, 10b; 10b*).

13. The method according to claim 12,
**characterized in that**
the actual position of the beam is determined from the measurements of the beam-position monitors (28; 28*) when including calibration data.

14. The method according to one of claims 12 through 13,
**characterized in that**
the beam-guide unit (10, 10a, 10b; 10b*) exhibits scanning magnets with correction function (32, 32*) for executing the raster scans, in which the control unit is proposed
such that first position signals are produced for executing a raster-scan therapy method and, with the aid of the deviations determined, second position signals are produced for adjusting the beam position, and that from the first position signals and the second position signals are produced third resultant position signals and they are transmitted to the scanning magnets with correction function (32, 32*) to execute the corrected raster-scan method.

15. The method according to one of claims 12 through 13,
**characterized in that**
the beam-guide unit (10, 10a, 10b; 10b*) exhibits scanning magnets (32, 32*) for executing the raster scans and correction magnets (30a, 30b), in particular made of lamellar iron, in which the control unit is proposed such that first position signals are produced for executing a raster-scan therapy method and, with the aid of the deviations determined, second position signals are produced for adjusting the beam position, and that the first position signals are transmitted to the scanning magnets (32; 32*) and the second position signals to the correction magnets (30a, 30b).

## Revendications

1. Système de thérapie par faisceau de particules (2) avec un équipement d'accélération (4) permettant de générer un faisceau de particules chargées,
avec une unité de guidage de faisceau (10, 10a, 10b; 10b*) pour guider le faisceau provenant de l'équipement (4) vers un site de traitement (18), dans lequel l'unité de guidage de faisceau (10, 10a, 10b; 10b*) comporte un portique pivotant avec au moins un écran de positionnement du faisceau (28; 28*) agencé sur le portique le long du trajet du faisceau (6) afin de déterminer la position transversale du faisceau et/ou la pulsation transversale au trajet du faisceau (6), et
avec une unité de commande communicant avec l'écran de positionnement du faisceau (28; 28*) et l'unité de guidage du faisceau (10, 10a, 10b; 10b*) pour déterminer les déviations de la position réelle par rapport à la position nominale du faisceau et pour la transmission des signaux de position pour ajuster la position du faisceau au niveau de l'unité de guidage du faisceau (10, 10a, 10b; 10b*), avec l'aide des déviations, dans lequel l'écran de positionnement du faisceau (28; 28*) est agencé sur la périphérie du faisceau perpendiculairement au trajet du faisceau (6) de sorte que la position du faisceau transversale et/ou la pulsation transversale par rapport au trajet du faisceau (6) est détectée par interaction directe avec les particules du faisceau dans la région de halo (36) dans au moins une dimension.

2. Système de thérapie par faisceau de particules selon la revendication 1,
**caractérisé en ce que**
l'écran de positionnement du faisceau (28; 28*) comporte une surface de détection orthogonale par rapport au trajet du faisceau (6), qui chevauche la section transversale du faisceau dans la région de halo (36).

3. Système de thérapie par faisceau de particules selon les revendications 1 ou 2, **caractérisé en ce que** l'unité de commande est proposée de telle sorte que la position réelle du faisceau est déterminée à partir des mesures de l'écran de positionnement du faisceau (28; 28*) lors de l'inclusion des données de calibration.

4. Système de thérapie par faisceau de particules selon l'une des revendications 1 à 3, **caractérisé en ce que** deux écrans de positionnement de faisceau (28; 28*) sont agencés l'un diamétralement opposé à l'autre sur la périphérie du faisceau.

5. Système de thérapie par faisceau de particules selon l'une des revendications 1 à 4, **caractérisé en ce que** l'écran de positionnement de faisceau (28; 28*) comporte au moins un compteur à scintillation (40; 46).

6. Système de thérapie par faisceau de particules selon la revendication 5, **caractérisé en ce que** l'équipement de scintillation a une épaisseur d'environ 0,5 à 2 mm dans la direction du trajet du faisceau (6).

7. Système de thérapie par faisceau de particules selon l'une des revendications 1 à 6, **caractérisé en ce que** l'écran de positionnement de faisceau (28; 28*) comporte au moins une chambre d'ionisation (44), préférablement cylindrique.

8. Système de thérapie par faisceau de particules selon l'une des revendications 1 à 7, **caractérisé en ce que** l'écran de positionnement de faisceau (28; 28*) est mobile transversalement par rapport au trajet de faisceau (6).

9. Système de thérapie par faisceau de particules selon l'une des revendications 1 à 8, **caractérisé en ce que** l'unité de guidage du faisceau (10, 10a, 10b; 10b*) comporte des aimants de balayage ayant une fonction de correction (32, 32*) pour exécuter des balayages de trame, durant lesquels l'unité de commande est proposée de telle sorte que des premiers signaux de position sont produits afin d'exécuter une méthode de thérapie par balayage de trame et, avec l'aide des déviations déterminées, des deuxièmes signaux de position sont produits pour ajuster la position du faisceau, et **en ce que**, à partir des premiers signaux de position et des deuxièmes signaux de position sont produits des troisièmes signaux de position résultants et qu'ils sont transmis aux aimants de balayage ayant fonction de correction (32, 32*) pour exécuter la méthode de balayage de trame corrigé.

10. Système de thérapie par faisceau de particules selon l'une des revendications 1 à 9, **caractérisé en ce que** l'unité de guidage de faisceau (10, 10a, 10b; 10b*) comporte des aimants de balayage (32, 32*) pour exécuter les balayages de trame et des aimants de correction (30a, 30b), en particulier en fer lamellaire, dans lequel l'unité de commande est proposée de telle sorte des premiers signaux de position sont produits pour réaliser une méthode de thérapie par balayage de trame et, à l'aide des déviations déterminées, des deuxièmes signaux de position sont produits pour ajuster la position du faisceau, et **en ce que** les premiers signaux de position sont transmis aux aimants de balayage (32; 32*) et les deuxièmes signaux de position aux aimants de correction (30a, 30b).

11. Système de thérapie par faisceau de particules selon l'une des revendications 1 à 11, **caractérisé en ce que** le collimateur magnéto-optique est inséré après l'écran de positionnement du faisceau (28; 28*) qui interagit directement avec les particules chargées du faisceau dans la région de halo (36) dans le trajet de faisceau (6).

12. Procédé comprenant :
la détermination des déviations dans la position réelle par rapport à la position nominale d'un faisceau de particules chargées généré par l'équipement d'accélération (4) du système de thérapie par faisceau de particules (2) et guidé par l'unité de guidage de faisceau (10, 10a, 10b; 10b*) du système de thérapie par faisceau de particules (2) provenant de l'équipement d'accélération (4) vers le site de traitement (18), basé sur la position du faisceau transversal déterminée par au moins un écran de positionnement de faisceau (28; 28*) agencé le long du trajet du faisceau (6) sur un portique pivotant de l'unité de guidage de faisceau (10, 10a, 10b; 10b*) pour la détermination de la position transversale du faisceau et/ou la pulsation transversale au trajet du faisceau (6), dans l'écran de positionnement du faisceau (28; 28*) est agencé sur la périphérie du faisceau perpendiculairement au trajet du faisceau (6) de sorte que la position transversale du faisceau et/ou de la pulsation transversale par rapport au trajet du faisceau (6) est détectée par l'interaction directe avec les particules du faisceau dans la région de halo (36) dans au moins une dimension, et
- la transmission des signaux de position vers l'unité de guidage du faisceau (10, 10a, 10b; 10b*) pour ajuster la position du faisceau, à l'aide des déviations, dans lequel la détermination des déviations et la transmission des signaux de position sont réalisées avec une unité de commande du système de thérapie par faisceau de particules en communication avec l'écran de positionnement du faisceau (28; 28*) et l'unité de guidage de faisceau (10, 10a, 10b; 10b*).

13. Procédé selon la revendication 12, **caractérisé en ce que** la position réelle du faisceau est déterminée à partie des mesures effectuées par les écrans de positionnement du faisceau (28; 28*) lors de l'inclusion des données de calibrage.

14. Procédé selon l'une des revendications 12 à 13, **caractérisé en ce que** l'unité de guidage du faisceau (10, 10a, 10b; 10b*) comporte des aimants de balayage ayant une fonction de correction (32, 32*) pour exécuter des balayages de trame, durant lesquels l'unité de commande est proposée de telle sorte que des premiers signaux de position sont produits afin de mettre en ceuvre une méthode de thérapie par balayage de trame et, avec l'aide des déviations déterminées, des deuxièmes signaux de position sont produits pour ajuster la position du faisceau, et **en ce que**, à partir des premiers signaux de position et des deuxièmes signaux de position sont produits des troisièmes signaux de position résultants et qu'ils sont transmis aux aimants de balayage ayant fonction de correction (32, 32*) pour exécuter la méthode de balayage trame corrigé.

15. Procédé selon l'une des revendications 12 à 13, **caractérisé en ce que** l'unité de guidage de faisceau (10, 10a, 10b; 10b*) comporte des aimants de balayage (32, 32*) pour exécuter les balayages de trame et des aimants de correction (30a, 30b), en particulier en fer lamellaire, dans lequel l'unité de commande est proposé de telle sorte que des premiers signaux de position sont produits pour réaliser une méthode de thérapie par balayage de trame et, à l'aide des déviations déterminées, des deuxièmes signaux de position sont produits pour ajuster la position du faisceau, et que les premiers signaux de position sont transmis aux aimants de balayage (32; 32*) et les deuxièmes signaux de position aux aimants de correction (30a, 30b).
